# EUROPÄISCHE PATENTSCHRIFT

(11) **EP 2 486 898 B1**
(45) Veröffentlichungstag und Bekanntmachung des Hinweises auf die Patenterteilung: **09.04.2014**
(21) Anmeldenummer: 12154697.2
(22) Anmeldetag: 09.02.2012
(51) Int. Cl.: A61F 5/042, A61G 13/10, A61G 13/12, A61B 17/60

(54) **Extensionsvorrichtung**
Extension device
Dispositif d'extension

(30) Priorität: 10.02.2011 DE 202011000308 U
(43) Veröffentlichungstag der Anmeldung: 15.08.2012
(73) Patentinhaber: MAQUET GmbH, 76437 Rastatt (DE)
(72) Erfinder: Koch, Guido, 76135 Karlsruhe (DE)
(74) Vertreter: Schaumburg, Thoenes, Thurn, Landskron, Eckert

(56) Entgegenhaltungen:
- WO-A1-2009/062324
- DE-U1-202009 013 905
- US-A- 5 645 079
- US-A1- 2007 265 635

## Beschreibung

Die Erfindung betrifft eine Extensionsvorrichtung, umfassend eine Patientenlagerfläche und einen Extensionsholm, der an seinem einen Ende über eine Schnittstelle mit der Patientenlagerfläche verbindbar ist und der an seinem anderen Ende eine Halterung für eine Zugspindelanordnung hat, wobei die Patientenlagerfläche mindestens zwei zueinander parallele Längsholme hat, die eine Beckenauflageplatte tragen.

Aufgabe einer Extensionsvorrichtung ist es, bei orthopädischen Eingriffen, wie z.B. Reposition von Knochenfrakturen im Beinbereich oder der Hüftendoprothetik, das Bein frei zugänglich zu lagern. Dazu wird der Fuß des Patienten in einem sogenannten Extensionsschuh aufgenommen, an dessen Sohle ein Zugspindelaggregat adaptiert ist. Über dieses Zugspindelaggregat kann in der Längsrichtung des Beines eine Zugkraft aufgebracht werden, um beispielsweise bei der Reposition die Bruchkanten des Knochens in ihre ursprüngliche Position zu bringen. Dabei soll das Bein nach innen und nach außen verschwenkbar sein.

Eine Extensionsvorrichtung der eingangs genannten Art ist beispielsweise aus dem deutschen Gebrauchsmuster 20 2009 013 905 U1 bekannt. Dabei ist die Patientenlagerfläche eines Operationstisches mit einem Orthopädieadapter gekoppelt, der in Verlängerung der Längsholme der Patientenlagerfläche Schnittstellen zum Ankoppeln eines Extensionsholmes hat. Der Extensionsholm hat hierzu ein Kupplungselement, mit dem er parallel zur Längsrichtung eines Längsholmes der Patientenlagerfläche an der Schnittstelle einhängbar ist. Das Kupplungselement hat ein Gelenk mit vertikaler Gelenkachse, um die der Extensionsholm in einer horizontalen Ebene verschwenkbar ist. Nachteilig an dieser Lösung ist, dass das Bedienelement für die Arretierung des Gelenkes in unmittelbarer Nähe der Beckenplatte unterhalb derselben und bei einer Hüftoperation somit im umnittelbaren Operationsbereich liegt. Dies macht die Bedienung schwierig, wenn das Bein des Patienten während der Operation verschwenkt werden soll.

Eine weitere Lösung ist aus der US 4,940,218 A bekannt. Diese Schrift zeigt einen orthopädischen Operationstisch mit zwei fest angelenkten Extensionsholmen, die sowohl in einer horizontalen Ebene als auch in einer vertikalen Ebene verschwenkbar sind. Die Extensionsholme können nicht gelöst werden.

Die US 2007/0265635 A1 zeigt eine Extensionsvorrichtung, umfassend eine Patientenlagerfläche und einen Extensionsholm, der an seinem einen Ende über eine Schnittstelle mit der Patientenlagerfläche verbindbar ist und eine Halterung für eine Zugspindelanordnung trägt, die entlang dem Extensionsholm verschiebbar ist. Die Schnittstelle umfasst ein holmseitiges Kupplungsteil, das über ein Kugelgelenk mit dem restlichen Extensionsholm verbunden ist und das in einen Sockel einsteckbar ist, der das lagerflächenseitige Kupplungsteil bildet und der Teil einer größeren Halterung ist, die über ein Joch mit den beiden Längsholmen der Patientenlagerfläche verbunden ist. Das Kugelgelenk ermöglicht ein Verschwenken des Extensionsholmes um eine vertikale Achse und eine horizontale Querachse und kann über eine vom Fußende des Extensionsholms her betätigbare Arretiervorrichtung arretiert werden.

Der Erfindung liegt die Aufgabe zugrunde, eine Extensionsvorrichtung der eingangs genannten Art anzugeben, die so ausgebildet ist, dass der Extensionsholm bei Bedarf bequem angekoppelt und abgekoppelt werden kann und darüber hinaus bequem verschwenkt werden kann, ohne dass dadurch der Operateur gestört oder die Sterilität des Operationsortes gefährdet wird.

Diese Aufgabe wird erfindungsgemäß dadurch gelöst, dass die Schnittstelle ein lagerflächenseitiges Kupplungsteil und ein mit dem Extensionsholm starr verbundenes holmseitiges Kupplungsteil umfasst, dass das lagerflächenseitige Kupplungsteil an einem der Längsholme der Patientenlagerfläche um eine zur Beckenauflageplatte senkrechte Achse schwenkbar gelagert ist und dass das lagerflächenseitige Kupplungsteil bezüglich einer Drehung um seine Schwenkachse gegenüber dem Längsholm mittels einer Arretiervorrichtung arretierbar ist, die über eine Fernbetätigung betätigbar ist.

Dadurch, dass das lagerflächenseitige Kupplungsteil an dem Längsholm der Lagerfläche schwenkbar gelagert ist, befindet sich das Gelenk für das Verschwenken des Extensionsholmes in der horizontalen Ebene auf der Seite der Patientenlagerfläche und nicht auf der Seite des Extensionsholmes. Dadurch kann das Gelenk sehr stabil ausgeführt werden, ohne dass das Gewicht des Extensionsholmes erhöht wird. Das Entfallen des Gelenkes auf der Seite des Extensionsholmes macht diesen leichter und damit auch bequemer zu handhaben. Die Fernbetätigung erlaubt es, beispielsweise vom Fußende des Patienten her die Arretiervorrichtung zu betätigen. Dadurch kann dies von einer Hilfsperson vorgenommen werden, die sich nicht im unmittelbaren sterilen Umfeld der Operationsstelle befindet. Dadurch braucht der Arzt für die Betätigung der Arretiervorrichtung nicht tätig zu werden und wird auch nicht durch andere Personen bei seiner Operationstätigkeit behindert.

Die Arretiervorrichtung kann beispielsweise ein am Längsholm angeordnetes Zahnsegment und ein zum Eingriff mit diesem bestimmtes, am lagerflächenseitigen Kupplungsteil angeordnetes Zahnsegment umfassen, wobei mindestens eines der Zahnsegmente in und außer Eingriff mit dem jeweils anderen Zahnsegment verstellbar ist. Vorzugsweise ist dabei das am Längsholm angeordnete Zahnsegment starr und das an dem Kupplungsteil angeordnete Zahnsegment beweglich. Die Arretiervorrichtung kann so ausgebildet sein, dass das bewegliche Zahnsegment in seine Eingriffstellung mit dem starren Zahnsegment vorgespannt und durch die Fernbetätigung in eine Freigabestellung ausrückbar ist. Die Fernbetätigung kann dabei über ein Druckmittel, also z.B. hydraulisch erfolgen. Bei einer bevorzugten Ausführungsform ist im holmseitigen Kupplungsteil ein druckmittelbetätigter Kolben so angeordnet, dass er bei eingekoppeltem Extensionsholm in Wirkverbindung mit der Arretiervorrichtung treten kann. In diesem Fall befindet sich also der Anschluss für das Druckmittel an dem holmseitigen Kupplungsteil.

Die Handhabung des Extensionsholmes beim Ankuppeln und Abkuppeln lässt sich weiter dadurch erleichtern, dass das lagerflächenseitige Kupplungsteil und das holmseitige Kupplungsteil so ausgebildet sind, dass die Fügerichtung beim Ankuppeln des Extensionsholmes im Wesentlichen horizontal und senkrecht zur Richtung der Längsholme der Patientenlagerfläche ist. Der Extensionsholm kann also von der Seite her angekoppelt werden, was wesentlich einfacher ist als wenn das Kupplungsteil des Extensionsholms unter dem bereits auf der Patientenlagerfläche liegenden Patienten in das lagerflächenseitige Kupplungsteil eingesteckt werden muss, wie dies beispielsweise bei der in dem deutschen Gebrauchsmuster 20 2009 013 905 U1 beschriebenen Lösung der Fall ist. Um eine sichere Kupplung und sichere Halterung des Extensionsholmes zu gewährleisten, hat das lagerflächenseitige Kupplungsteil bei einer bevorzugten Ausführungsform der Erfindung eine seitlich nach außen offene Aufnahmetasche zur Aufnahme des holmseitigen Kupplungsteiles. Die Anordnung ist dabei so getroffen, dass das holmseitige Kupplungsteil in seiner Kupplungsstellung an dem lagerflächenseitigen Kupplungsteil verriegelbar ist.

Um zum Beispiel die Reposition von Knochenfrakturen mit Hilfe der Extensionsvorrichtung überprüfen zu können, ist es häufig erforderlich, das betreffende Bein des Patienten zu röntgen. Dabei darf der Extensionsholm die Bilderzeugung nicht behindern. Zu diesem Zweck ist der Extensionsholm in an sich bekannter Weise aus einem röntgenfähigen Material, beispielsweise einem Faserverbundstoff hergestellt. Dieses Material erlaubt es auch, dem Extensionsholm eine solche Form zu geben, dass er die Durchstrahlung des Patientenbeines nicht oder so gering wie möglich behindert. So kann der Extensionsholm in seiner Länge abgekröpft sein mit einem an das holmseitige Kupplungsteil anschließenden ersten geraden Abschnitt, der im angekoppelten Zustand parallel zu dem Längsholm der Patientenlagerfläche gerichtet ist, einem gegenüber dem ersten Abschnitt abgebogenen Übergangsabschnitt und einem zum ersten Abschnitt wiederum parallelen zweiten geraden Abschnitt. Dadurch wird der Extensionsholm bezogen auf ein Patientenbein zur Seite hin ausgelenkt, so dass er bei einem Durchstrahlen des Patientenbeines in vertikaler Richtung nicht im Bild erscheint.

Bei einer besonders bevorzugten Ausführungsform der Erfindung ist der Extensionsholm in Form eines Rohres mit ovalem Querschnitt ausgebildet, wobei der Extensionsholm relativ zu dem holmseitigen Kupplungsteil so angeordnet ist, dass im angekuppelten Zustand des Extensionsholmes die längere Querschnittsachse des Ovals gegenüber der Horizontalen geneigt ist. Die ovale Querschnittsform und die Orientierung des Ovals führen dazu, dass bei einem von der Vertikalen abweichenden Durchstrahlen des Patientenbeines, bei dem der Extensionsholm ins Bild gelangen kann, das durchstrahlte Material des Extensionsholmes maximal der doppelten Wandstärke des Holmes entspricht. Bei einem quadratischen Querschnitt des Extensionsholmes dagegen kann es geschehen, dass ein Teil der Strahlen über eine größere Länge innerhalb der Holmwand verläuft, so dass dieser Teil der Strahlen stark von dem Holmmaterial absorbiert wird. Zweckmäßigerweise ist die Halterung für das Zugspindelaggregat an dem der Patientenlagerfläche fernen Ende des Extensionsholmes mit diesem durch ein Viergelenkgestänge verbunden, dessen Gelenkachsen im angekoppelten Zustand des Extensionsholmes horizontal und quer zur Längsrichtung des Extensionsholmes gerichtet sind. Das Viergelenkgestänge erlaubt es, das Zugspindelaggregat und damit das Patientenbein auf einer vertikalen Kreisbahn um das Hüftgelenk des Patienten zu bewegen, ohne dass auf das Patientenbein ein Zug oder ein Schub ausgewirkt wird.

Die folgende Beschreibung erläutert in Verbindung mit den beigefügten Zeichnungen die Verbindung anhand eines Ausführungsbeispieles. Es zeigen:
- Fig.1: eine teilweise schematische perspektivische Ansicht eines Teils einer Patientenlagerfläche mit angekoppeltem Extensionsholm und Zugspindelaggregat,
- Fig.2: eine Seitenansicht der Extensionsvorrichtung nach Fig.1 ohne Zugspindelaggregat,
- Fig.3: eine der Fig.2 entsprechende Ansicht der Extensionsvorrichtung mit einer verschwenkten Halterung für das Zugspindelaggregat,
- Fig.4: eine vergrößerte perspektivische Darstellung eines Lagerflächenholmes allein mit der Schnittstelle für den angekoppelten Extensionsholm,
- Fig.5: eine perspektivische, teilweise schematische Darstellung des lagerflächenseitigen Kupplungsteiles,
- Fig.6: eine Ansicht des lagerflächenseitigen Kupplungsteiles in Richtung des Pfeiles A in Fig.5,
- Fig.7: eine Draufsicht auf das lagerflächenseitige Kupplungsteil,
- Fig.8: eine Draufsicht auf das holmseitige Kupplungsteil,
- Fig.9: eine Ansicht des holmseitigen Kupplungsteiles in Richtung des Pfeiles B in Fig.8,
- Fig.10: einen Schnitt durch den Lagerflächenholm und die Schnittstelle entlang der Linie X-X in Fig.4,
- Fig.11: einen Steilschnitt durch die Schnittstelle entlang der Linie XI-XI in Fig.4 und
- Fig.12: einen Querschnitt durch den Extensionsholm entlang der Linie XII-XII in Fig.1.

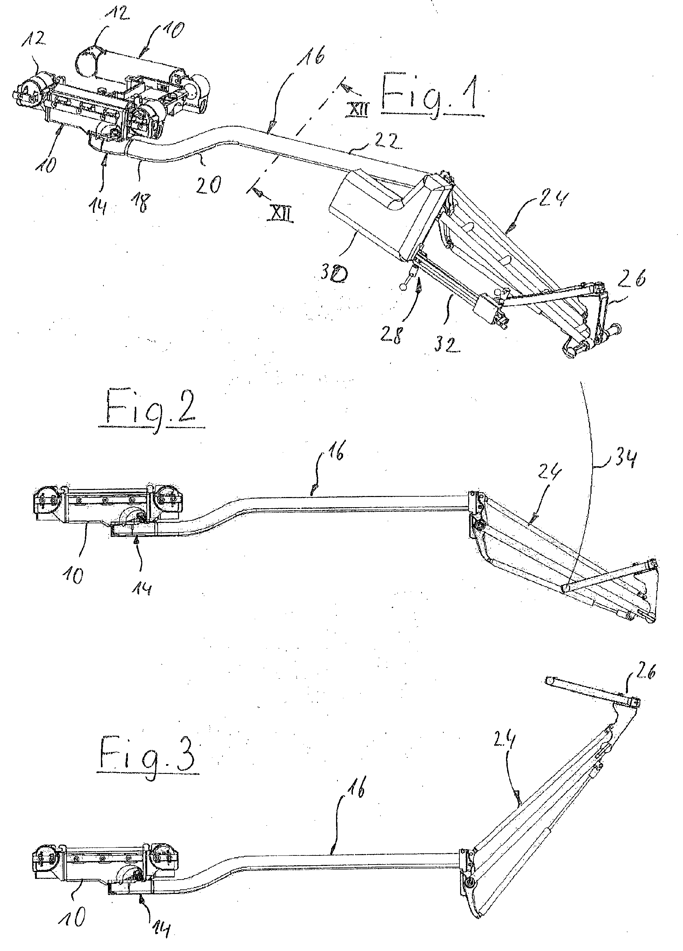

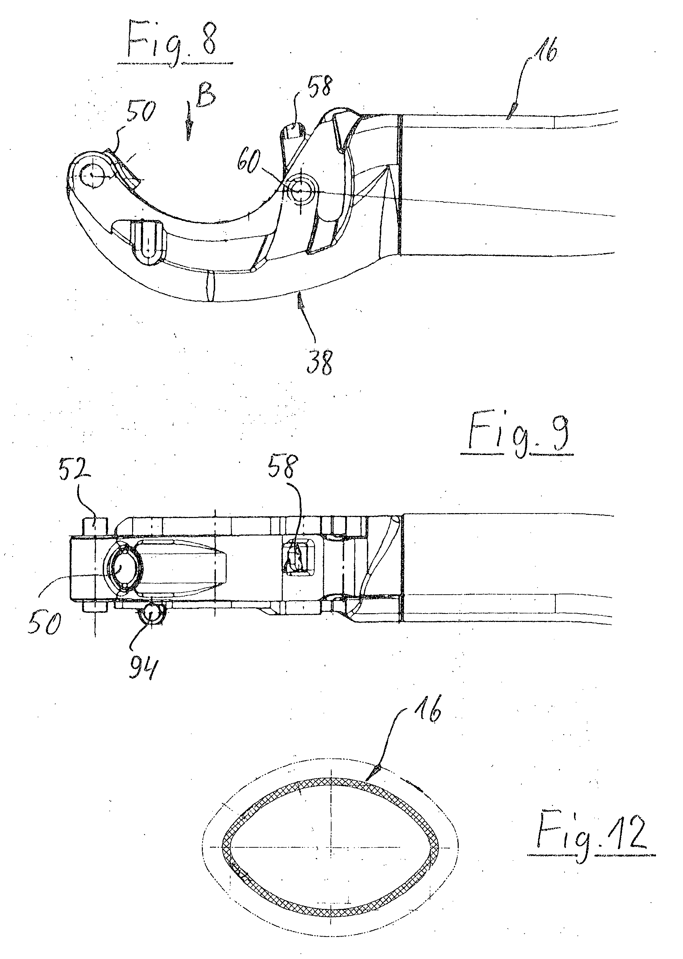

Die in der Fig.1 dargestellte Extensionsvorrichtung umfasst einen schematisch dargestellten Teil einer Patientenlagerfläche mit zwei zueinander parallelen Lagerflächenholmen 10, die an ihren Längsenden jeweils Anschlusselemente 12 zum Anstecken weiterer Lagerflächenteile haben. Die Lagerflächenholme 10 tragen eine nicht dargestellte Beckenplatte zur Lagerung eines Patienten. Mit einem der Lagerflächenholme 10 ist an einer Schnittstelle 14 ein allgemein mit 16 bezeichneter Extensionsholm gekoppelt. Der Extensionsholm ist von einem abgekröpften Rohr gebildet, das einen ersten der Lagerfläche nahen geraden Abschnitt 18, einen gebogenen Übergangsabschnitt 20 und einen zum ersten Abschnitt 18 parallelen zweiten Abschnitt 22 umfasst. Das Rohr ist aus Karbonfaserverbundmaterial hergestellt und hat einen ovalen Querschnitt, der in Fig.12 wiedergegeben ist.

An seinem dem Lagerflächenabschnitt fernen Ende ist der Extensionsholm mit einem Viergelenkgestänge 24 verbunden, das eine Halterung 26 für ein Zugspindelaggregat 28 trägt. Das Zugspindelaggregat umfasst einen Schuh 30 zur Aufnahme eines Patientenfußes sowie ein Zuggestänge 32, mit dessen Hilfe ein Zug auf das Patientenbein ausgeübt werden kann. Mit Hilfe des Viergelenk- oder Parallelogrammgestänges 24 kann der Verbindungspunkt der Halterung 26 mit dem Zugspindelaggregat 28 auf einer Kreisbahn 34 bewegt werden (Fig.2), deren Mittelpunkt das Hüftgelenk des Patienten ist. Dadurch kann das Bein des Patienten im Hüftgelenk nach oben und unten verschwenkt werden, ohne dass durch das Verschwenken ein Zug auf das Patientenbein ausgeübt wird.

Im Folgenden soll die Schnittstelle für die Kopplung des Extensionsholmes 16 mit dem Lagerflächenholm 10 näher erläutert werden.

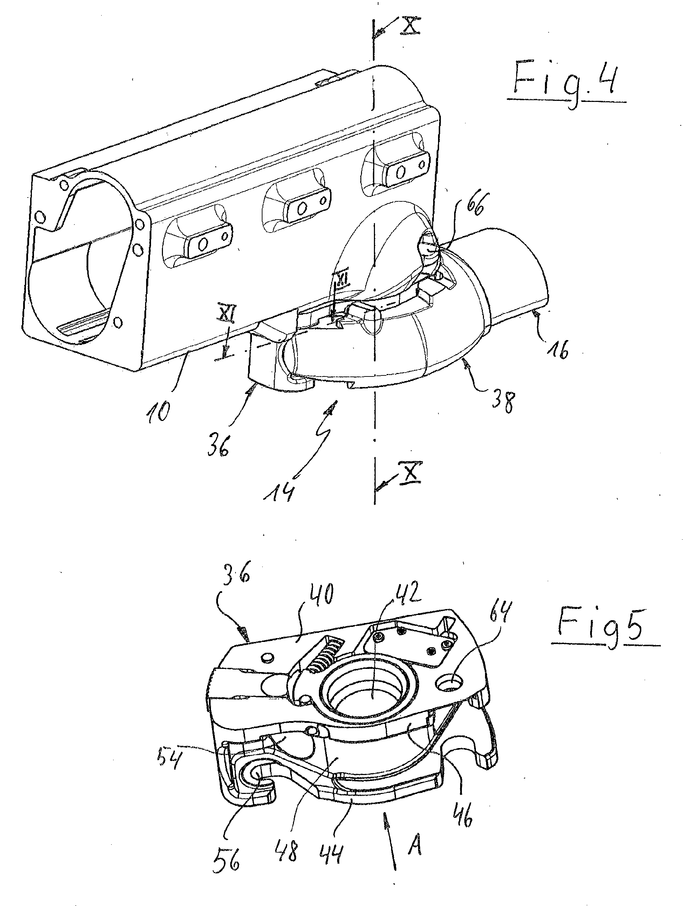

Fig.4 zeigt den Extensionsholm 10 mit der Schnittstelle 14, die ein lagerflächenseitiges Kupplungsteil 36 und ein mit dem Extensionsholm 16 verbundenes holmseitiges Kupplungsteil 38 umfasst. Das lagerflächenseitige Kupplungsteil ist in den Fig.5 bis 7 näher dargestellt. Es ist in Form eines blockförmigen Gehäuses 40 ausgebildet, das in seiner Mitte eine durchgehende Lageröffnung 42 hat, mit dem das Kupplungsteil 36 auf einem an der Unterseite des Längsholmes 10 angeordneten, nicht dargestellten starren Zapfen um die Zapfenachse schwenkbar gelagert ist. Dieses Zapfenlager ermöglicht das Verschwenken des lagerflächenseitigen Kupplungsteiles 36 und damit des an ihm angekoppelten Extensionsholmes 16 um eine vertikale Achse.

An seiner einen Seite hat das blockförmige Gehäuse 40 eine von einem unteren Flansch 44 und einem oberen Flansch 46 begrenzte Aufnahmetasche 48 zur Aufnahme des holmseitigen Kupplungsteiles 38, das in den Fig.8 und 9 dargestellt ist. Es ist sichelförmig ausgebildet und hat an seinem freien Sichelende innen einen flachen zylindrischen Fortsatz 50 (Fig.8) sowie einen Zapfen 52, die zum Eingriff in eine entsprechende kreisförmige Aussparung 54 am Grunde der Tasche 48 bzw. in eine Hakenöffnung 56 bestimmt sind, die in dem unteren Flansch 44 ausgebildet ist. An seinem dem Extensionsholm 16 nahen Ende hat das sichelförmig gekrümmte Kupplungsteil 38 eine Rastklinke 58, die so in dem Kupplungsteil gelagert ist, dass sie durch Druck auf einen Betätigungsstift 60 zwischen einer Sperrstellung und einer Freigabestellung verschwenkt werden kann. Die Rastklinke 58 ist zum

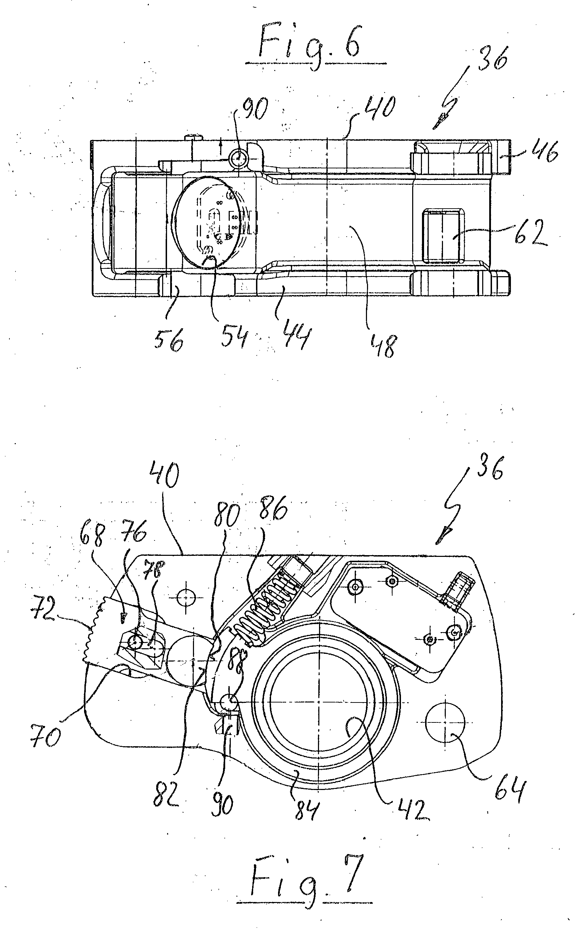

Eingriff in eine Rastöffnung 62 bestimmt, die am Grund der Aufnahmetasche 48 in dem lagerflächenseitigen Kupplungsteil 36 ausgebildet ist (Fig.6).

Zum Koppeln der beiden Kupplungsteile 36 und 38 wird das holmseitige Kupplungsteil 38 mit dem Zapfen 52 in die Hakenöffnung 56 an dem Flansch 44 des lagerflächenseitigen Kupplungsteiles 36 eingeführt, wobei auch der zylindrische Fortsatz 50 in die Öffnung 54 am Grunde der Aufnahmetasche 48 eintritt. Dann wird der Extensionsholm 16 zusammen mit dem Kupplungsteil 38 um die Achse des Zapfens 52 so verschwenkt, dass das Kupplungsteil 38 in die Aufnahmetasche 48 eintritt und die Rastklinke 58 in der Rastöffnung 62 einrastet. Dann liegt der Betätigungsstift 60 in Flucht mit einer Bohrung 64, die im oberen Flansch 46 des Gehäuses 40 ausgebildet ist. Durch diese Bohrung 64 hindurch kann der Betätigungsstift 60 mittels einer an dem Lagerflächenholm 10 angeordneten Drucktaste 66 betätigt werden, um die Rastklinke 58 wieder in ihre Freigabestellung zu überführen. Wie man in Fig.4 erkennt, ist die Anordnung so getroffen, dass die Bedienungsperson das holmseitige Kupplungsteil 38 mit der Hand untergreifen und mit dem Daumen die Drucktaste 66 betätigen kann, so dass beim Lösen der Verriegelung durch Niederdrücken der Drucktaste 66 gleichzeitige der Extensionsholm 16 sicher gefasst werden und nicht aus dem lagerflächenseitigen Kupplungsteil 36 herausfallen kann.

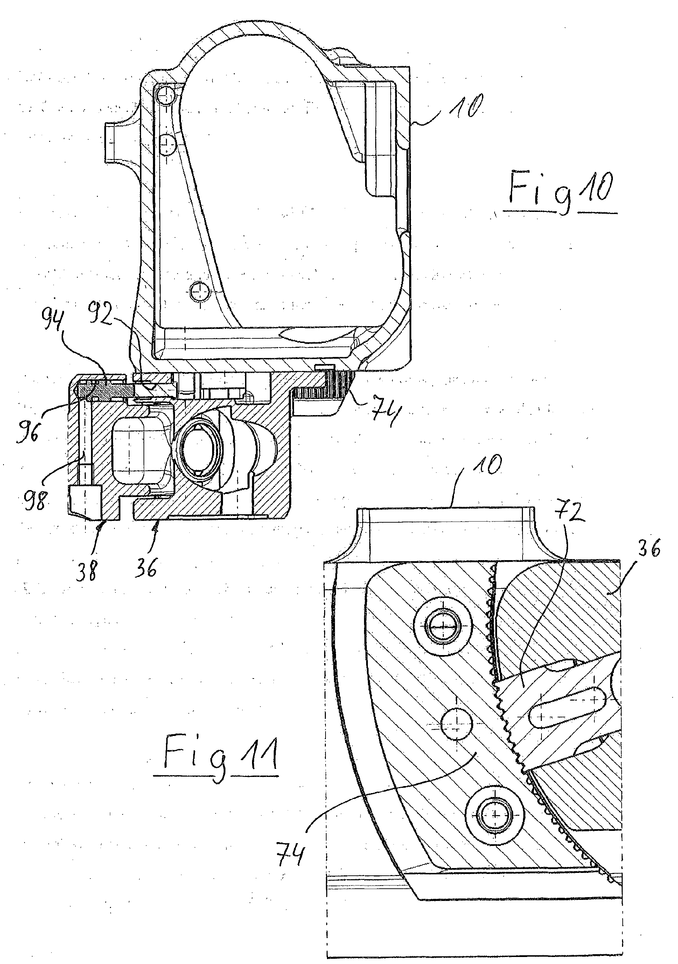

Wie man in den Fig.5 und 7 erkennt, ist in dem oberen Flansch 46 des Gehäuses 40 ein Schieber 68 in einer im Wesentlichen radial zu der Durchtrittsöffnung 42 verlaufenden flachen Nut 70 verschiebbar gelagert. Der Schieber hat an seinem der Durchtrittsöffnung 42 fernen Ende ein Zahnsegment 72, das zum Eingriff mit einem starr mit dem Längsholm 10 der Patientenlagerfläche verbundenen Zahnsegment 74 bestimmt ist (wie dies Fig.11 zeigt), um das lagerflächenseitige Kupplungsteil 36 in einer bestimmten Schwenkstellung um die Vertikalachse zu arretieren. Der Schieber 68 wird mittels eines gehäusefesten Stiftes 76, der in einen Schlitz 78 des Schiebers eingreift, geführt. Ferner liegt der Schieber über eine Nockenkurve 80 an einem Nocken 82 an, der mit einem Ring 84 verbunden ist, der seinerseits um die Lagerachse des lagerflächenseitigen Kupplungsteiles 36 drehbar gelagert ist, wie dies insbesondere Fig.7 zeigt. Der Ring 84 wird durch eine Schraubendruckfeder 86 in der Fig.7 im Gegenuhrzeigersinn vorgespannt und liegt mit der Flanke des Nockens 82 über eine Rolle 88 an einem Betätigungsbolzen 90 an, der in einer Bohrung 92 (Fig.10) des Gehäuses 40 verschiebbar angeordnet ist. Wird der Bolzen 90 in Fig.7 nach oben verschoben und damit der Ring 84 in der Fig.7 gegen den Druck der Schraubendruckfeder 86 im Uhrzeigersinn verschwenkt, bewegt sich der Schieber 68 unter der Wirkung einer nicht dargestellten Feder in Richtung auf das Zentrum der Durchtrittsöffnung 42, so dass das Zahnsegment 72 außer Eingriff mit dem Zahnsegment 74 kommt. Dann kann das Gehäuse 40, d.h. das lagerflächenseitige Kupplungsteil 36 um die Vertikalachse verschwenkt werden, bis der Bolzen 90 wieder freigegeben wird und der Schieber 68 unter der Wirkung des im Gegenuhrzeigersinn rotierenden Nockens 82 nach außen in Eingriff mit dem Zahnsegment 74 gedrückt wird.

Die Betätigung des Bolzens 90 erfolgt in dem dargestellten Ausführungsbeispiel hydraulisch mit Hilfe eines Kolbens 94, der in einer zylindrischen Bohrung 96 des holmseitigen Kupplungsteiles 38 so angeordnet ist, dass er beim Koppeln der beiden Kupplungsteile 36, 38 in Flucht mit dem Betätigungsbolzen 90 tritt. Der Kolben 94 kann durch ein Hydraulikfluid bewegt werden, das durch einen in dem holmseitigen Kupplungsteil 38 ausgebildeten Fluidkanal 98 zugeführt werden kann. Damit ist eine Fernbetätigung der Arretiervorrichtung 72, 74 möglich.

## Patentansprüche

1. Extensionsvorrichtung, umfassend eine Patientenlagerfläche und einen Extensionsholm (16), der an seinem einen Ende über eine Schnittstelle (14) mit der Patientenlagerfläche verbindbar ist und an seinem anderen Ende eine Halterung (26) für eine Zugspindelanordnung (28) hat, wobei die Patientenlagerfläche mindestens zwei zueinander parallele Längsholme (10) hat, die eine Beckenauflageplatte tragen, **dadurch gekennzeichnet, dass** die Schnittstelle (14) ein lagerflächenseitiges Kupplungsteil (36) und ein mit dem Extensionsholm (16) starr verbundenes holmseitiges Kupplungsteil (38) umfasst, dass das lagerflächenseitige Kupplungsteil (36) an einem der Längsholme (10) um eine zur Beckenauflageplatte senkrechte Achse schwenkbar gelagert ist und dass das lagerflächenseitige Kupplungsteil (36) bezüglich einer Drehung um seine Schwenkachse gegenüber dem Längsholm (10) mittels einer Arretiervorrichtung (72, 74) arretierbar ist, die über eine Fernbetätigung (94, 90) betätigbar ist.

2. Extensionsvorrichtung nach Anspruch 1, **dadurch gekennzeichnet, dass** die Arretiervorrichtung ein am Längsholm (10) angeordnetes Zahnsegment (74) und ein zum Eingriff mit diesem bestimmtes, am lagerflächenseitigen Kopplungsteil (36) angeordnetes Zahnsegment (72) umfasst, wobei mindestens eines der Zahnsegmente (72, 74) in und außer Eingriff mit dem jeweils anderen Zahnsegment (74, 72) verstellbar ist.

3. Extensionsvorrichtung nach Anspruch 2, **dadurch gekennzeichnet, dass** das am Längsholm (10) angeordnete Zahnsegment (74) starr und das an dem Kupplungsteil (36) angeordnete Zahnsegment (72) beweglich ist.

4. Extensionsvorrichtung nach Anspruch 2 oder 3, **dadurch gekennzeichnet, dass** das bewegliche Zahnsegment (72) in seine Eingriffstellung mit dem starren Zahnsegment (74) vorgespannt und durch die Fernbetätigung in eine Freigabestellung ausrückbar ist.

5. Extensionsvorrichtung nach einem der Ansprüche 1 bis 4, **dadurch gekennzeichnet, dass** die Fernbetätigung über ein Druckfluid erfolgt.

6. Extensionsvorrichtung nach einem der Ansprüche 1 bis 5, **dadurch gekennzeichnet, dass** das lagerflächenseitige Kupplungsteil (36) und das holmseitige Kupplungsteil (38) so ausgebildet sind, dass die Fügerichtung beim Ankuppeln des Extensionsholmes (16) im Wesentlichen horizontal und senkrecht zur Richtung der Längsholme (10) der Patientenlagerfläche ist.

7. Extensionsvorrichtung nach Anspruch 6, **dadurch gekennzeichnet, dass** das lagerflächenseitige Kupplungsteil (36) eine seitlich nach außen offen Aufnahmetasche (48) zur Aufnahme des holmseitigen Kupplungsteiles (38) hat.

8. Extensionsvorrichtung nach einem der Ansprüche 1 bis 7, **dadurch gekennzeichnet, dass** das holmseitige Kupplungsteil (38) in seiner Kupplungsstellung an dem lagerflächenseitigen Kupplungsteil (36) verriegelbar ist.

9. Extensionsvorrichtung nach einem der Ansprüche 1 bis 8, **dadurch gekennzeichnet, dass** der Extensionsholm (16) aus einem röntgenfähigen Material besteht.

10. Extensionsvorrichtung nach Anspruch 9, **dadurch gekennzeichnet, dass** der Extensionsholm (16) aus einem Faserverbundstoff besteht.

11. Extensionsvorrichtung nach Anspruch 9 oder 10, **dadurch gekennzeichnet, dass** der Extensionsholm (16) in Form eines Rohres mit ovalem Querschnitt ausgebildet ist.

12. Extensionsvorrichtung nach Anspruch 11, **dadurch gekennzeichnet, dass** der Extensionsholm (16) relativ zum holmseitigen Kupplungsteil (38) so ausgerichtet ist, dass im eingekuppelten Zustand die längere Querschnittsachse des Ovals gegenüber der Horizontalen geneigt ist.

13. Extensionsvorrichtung nach einem der Ansprüche 9 bis 12, **dadurch gekennzeichnet, dass** der Extensionsholm (16) abgekröpft ist mit einem an dem holmseitigen Kupplungsteil (38) anschließenden ersten geraden Abschnitt (18), der im angekoppelten Zustand parallel zu den Längsholmen (10) der Patientenlagerfläche gerichtet ist, einem gegenüber dem ersten Abschnitt abgebogenen Übergangsabschnitt (20) und einem zum ersten Abschnitt (18) parallelen zweiten geraden Abschnitt (22).

14. Extensionsvorrichtung nach einem der Ansprüche 1 bis 13, **dadurch gekennzeichnet, dass** die Halterung (26) für das Zugspindelaggregat mit dem Extensionsholm (16) über ein Viergelenkgestänge (24) verbunden ist, dessen Gelenkachse im angekoppelten Zustand des Extensionsholmes (16) horizontal und quer zur Längsrichtung des Extensionsholmes (16) gerichtet ist.

15. Extensionsvorrichtung nach einem der Ansprüche 1 bis 14, **dadurch gekennzeichnet, dass** im holmseitigen kupplungsteil (38) ein druckmittelbetäligter Kolben (94) so angeordnet ist, dass er im eingekoppelten Zustand des Exiensionsholmes (16) in Wirkverbindung mit der Arretiervorrichtung treten kann.

## Claims

1. An extension device comprising a patient support surface and an extension beam (16) that can be connected on its one end via an interface (14) to the patient support surface and that has a holder (26) for a feed rod arrangement (28) on its other end, whereby the patient support surface has at least two longitudinal beams (10) parallel to each other that carry a pelvic support plate, **characterized in that** the interface (14) comprises a coupling part (36) on the support surface side and comprises a coupling part (38) on the beam side which is rigidly connected to the extension beam (16), that the coupling part (36) on the support surface side is supported on one of the longitudinal beams (10) in such a manner that it can pivot about an axis perpendicular to the pelvic support plate, and that the coupling part (36) on the support surface side can be locked by a locking device (72, 74) against a rotation about its pivot axis relative to the longitudinal beam (10), which locking device can be actuated by a remote actuation (94, 90).

2. The extension device according to Claim 1, **characterized in that** the locking device comprises a toothed segment (74) arranged on the longitudinal beam (10) and a toothed segment (72) arranged on the coupling part (36) on the support surface side for engaging with the first toothed segment, whereby at least one of the toothed segments (72, 74) can be adjusted in and out of engagement with the particular other toothed segment (74, 72).

3. The extension device according to Claim 2, **characterized in that** the toothed segment (74) arranged on the longitudinal beam (10) is rigid and that the toothed segment (72) arranged on the coupling part (36) is movable.

4. The extension device according to Claim 2 or 3, **characterized in that** the movable toothed segment (72) is biased into its engagement position with the rigid toothed segment (74) and can be moved out into a release position by the remote actuation.

5. The extension device according to one of Claims 1 to 4, **characterized in that** the remote actuation takes place via a pressure fluid.

6. The extension device according to one of Claims 1 to 5, **characterized in that** the coupling part (36) on the support surface side and the coupling part (38) on the beam side are constructed in such a manner that the joining direction during the coupling of the extension beam (16) is substantially horizontal and perpendicular to the direction of the longitudinal beams (10) of the patient support surface.

7. The extension device according to Claim 6, **characterized in that** the coupling part (36) on the support surface side has a receiving pocket (48) laterally open to the outside for receiving the coupling part (38) on the beam side.

8. The extension device according to one of Claims 1 to 7, **characterized in** hat the coupling part (38) on the beam side can be locked in its coupling position on the coupling part (36) on the support surface side.

9. The extension device according to one of Claims 1 to 8, **characterized in that** the extension beam (16) consists of a material capable of being X-rayed.

10. The extension device according to Claim 9, **characterized in that** the extension beam (16) consists of a fibrous composite material.

11. The extension device according to Claim 9 or 10, **characterized in that** the extension beam (16) is constructed in the shape of a tube with an oval cross section.

12. The extension device according to Claim 11, **characterized in that** the extension beam (16) is aligned relative to the coupling part (38) on the beam side in such a manner that in the coupled state the longer cross-sectional axis of the oval is inclined relative to the horizontal.

13. The extension device according to one of Claims 9 to 12, **characterized in that** the extension beam (16) is cranked with a first straight section (18) following the coupling part (38) on the beam side which first section is aligned in the coupled state in parallel to the longitudinal beams (10) of the patient support surface, with a transitional section (20) bent relative to the first section, and with a second straight section (22) which is parallel to the first section (18).

14. The extension device according to one of Claims 1 to 13, **characterized in that** the holder (26) for the feed rod unit is connected to the extension beam (16) by a linkage (24) with four articulations whose articulation axis is directed in the coupled state of the extension beam (16) horizontally and transversely to the longitudinal direction of the extension beam (18).

15. The extension device according to one of Claims 1 to 14, **characterized in that** a piston (94) actuated by pressure means is arranged in the coupling part (38) on the beam side in such a manner that it can enter into an active connection with the locking device when the extension beam (16) is coupled in.

## Revendications

1. Dispositif d'extension, comprenant une surface de support pour patient et une barre d'extension (16) qui peut être reliée par une de ses extrémités à la surface de support pour patient au moyen d'un accouplement (14), et qui présente à son autre extrémité un élément de retenue (26) pour un ensemble broche de traction (28), la surface de support pour patient présentant au moins deux longerons (10) parallèles l'un à l'autre qui portent une plaque d'appui pour le bassin, **caractérisé en ce que** l'accouplement (14) comprend une partie d'accouplement côté surface de support (36) et une partie d'accouplement côté barre (38) reliée rigidement à la barre d'extension (16), **en ce que** la partie d'accouplement côté surface de support (36) est montée sur un des longerons (10) de manière à pouvoir pivoter par rapport à un axe perpendiculaire à la plaque de support pour bassin, et **en ce que** la partie d'accouplement côté surface de support (36) peut être empêchée de tourner autour de son axe de pivotement par rapport au longeron (10) au moyen d'un dispositif d'arrêt (72, 74), qui peut être actionné par un dispositif d'actionnement à distance (94, 90).

2. Dispositif d'extension selon la revendication 1, **caractérisé en ce que** le dispositif d'arrêt comprend un segment denté (74) disposé sur le longeron (10) et un segment denté (72) disposé sur la partie d'accouplement côté surface de support (36) et conçu pour s'engrener avec ledit segment denté (74), au moins un des segments dentés (72, 74) pouvant être amené en prise avec l'autre segment denté (74, 72) et s'en dégager.

3. Dispositif d'extension selon la revendication 2, **caractérisé en ce que** le segment denté (74) disposé sur le longeron (10) est fixe et le segment denté (72) disposé sur la partie d'accouplement (36) est mobile.

4. Dispositif d'extension selon la revendication 2 ou 3, **caractérisé en ce que** le segment denté mobile (72), lorsqu'il se trouve dans sa position d'engagement, est précontraint avec le segment denté (74) fixe et peut être dégagé dans une position de libération par le dispositif d'actionnement à distance.

5. Dispositif d'extension selon l'une quelconque des revendications 1 à 4, **caractérisé en ce que** l'actionnement à distance s'effectue au moyen d'un fluide sous pression.

6. Dispositif d'extension selon l'une quelconque des revendications 1 à 5, **caractérisé en ce que** la partie d'accouplement côté surface de support (36) et la partie d'accouplement côté barre (38) sont réalisées de sorte que la direction d'assemblage, lors de l'accouplement de la barre d'extension (16), est sensiblement horizontale et perpendiculaire à la direction des longerons (10) de la surface de support pour patient.

7. Dispositif d'extension selon la revendication 6, **caractérisé en ce que** la partie d'accouplement côté surface de support (36) présente une poche réceptrice (48) ouverte latéralement vers l'extérieur destinée à recevoir la partie d'accouplement côté barre (38).

8. Dispositif d'extension selon l'une quelconque des revendications 1 à 7, **caractérisé en ce que** la partie d'accouplement côté barre (38), lorsqu'elle se trouve dans sa position d'accouplement, peut être verrouillée sur la partie d'accouplement côté surface de support (36).

9. Dispositif d'extension selon l'une quelconque des revendications 1 à 8, **caractérisé en ce que** la barre d'extension (16) est constituée d'un matériau radio-opaque.

10. Dispositif d'extension selon la revendication 9, **caractérisé en ce que** la barre d'extension (16) est constituée d'un matériau composite renforcé par des fibres.

11. Dispositif d'extension selon la revendication 9 ou 10, **caractérisé en ce que** la barre d'extension (16) est réalisée sous forme d'un tube présentant une section transversale ovale.

12. Dispositif d'extension selon la revendication 11, **caractérisé en ce que** la barre d'extension (16) est orientée par rapport à la partie d'accouplement côté barre (38), de sorte qu'une fois l'accouplement mis en oeuvre, le plus grand axe de section transversale de l'ovale est incliné par rapport à l'horizontale.

13. Dispositif d'extension selon l'une quelconque des revendications 9 à 12, **caractérisé en ce que** la barre d'extension (16) est coudée et est pourvue d'une première partie rectiligne (18) adjacente à la partie d'accouplement côté barre (38), laquelle, dans l'état accouplé, est orientée parallèlement aux longerons (10) de la surface de support pour patient, d'une partie de transition (20) recourbée par rapport à la première partie et d'une seconde partie rectiligne (22) parallèle à la première partie (18).

14. Dispositif d'extension selon l'une quelconque des revendications 1 à 13, **caractérisé en ce que** l'élément de retenue (26) pour l'ensemble de broche de traction est relié à la barre d'extension (16) par un quadrilatère articulé (24) dont l'axe d'articulation, une fois la barre d'extension (16) accouplée, est orienté horizontalement et transversalement à la direction longitudinale de la barre d'extension (16).

15. Dispositif d'extension selon l'une quelconque des revendications 1 à 14, **caractérisé en ce qu'**un piston (94) actionné par un fluide sous pression est agencé dans la partie d'accouplement côté barre (38), de sorte que, une fois la barre d'extension (16) accouplée, il peut coopérer avec le dispositif d'arrêt.
